Europäisches Patentamt
European Patent Office
Office européen des brevets

(11) Publication number: **0 204 401**
**A1**

# EUROPEAN PATENT APPLICATION

(21) Application number: **86302560.7**

(22) Date of filing: **07.04.86**

(51) Int. Cl.⁴: **C 12 N 15/00**
**C 12 P 21/00, C 07 K 15/00**
**C 07 K 13/00, C 07 H 21/04**
**C 12 N 1/20, C 12 N 1/16**
**C 12 N 1/14, C 12 N 5/00**
**//(C12R1/19, 1:38, 1:07)**

(30) Priority: **09.04.85 US 721293**

(43) Date of publication of application:
**10.12.86 Bulletin 86/50**

(84) Designated Contracting States:
**AT BE CH DE FR GB IT LI LU NL SE**

(71) Applicant: **BIOGEN N.V.**
**15 Pietermaai**
**Willemstad Curacao, Netherlands Antilles(NL)**

(72) Inventor: **Panayotatos, Nikos**
**17 Pre-Jerome**
**1205 Geneva(CH)**

(74) Representative: **Bannerman, David Gardner et al,**
**Withers & Rogers 4 Dyer's Buildings Holborn**
**London, EC1N 2JT(GB)**

(54) **Method of improving the yield of polypeptides produced in a host cell by stabilizing mRNA.**

(57) A method of improving the yield of a desired polypeptide produced in a host cell comprising culturing a host cell transformed with a DNA sequence coding on expression for that protein, said DNA sequence also comprising an RNase III site downstream of said coding sequence, said site being such that when said site is cleaved by RNase III, a folded structure remains that stabilizes the mRNA transcript for the desired protein relative to uncleaved mRNA, is disclosed.

## METHOD OF IMPROVING THE YIELD OF POLYPEPTIDES PRODUCED IN A HOST CELL BY STABILIZING MESSENGER RNA

The present invention relates to a method of stabilizing mRNA, and thus improving the yield of polypeptides produced in a host cell.  The method of stabilizing mRNA of the present invention is especially useful when the stability of the mRNA is a  .  rate limiting factor in the production of the desired polypeptides.

## Background Art

Recombinant DNA techniques have made it possible to prepare proteins and polypeptides, such as interferons and various hormones, that were heretofore unavailable in significant amounts, by culturing host cells transformed with a DNA sequence coding for those proteins or polypeptides and isolating the produced protein.  Although much progress has been made in obtaining significant amounts of proteins relatively inexpensively, there is considerable room for improvement.

The level of production of a protein in a host cell is governed by three major factors:  the number of copies of its gene within the cell, the efficiency with which those gene copies are transcribed and the efficiency with which the resultant messenger RNA ("mRNA") is translated.  Optimization of each of these factors is desirable if a protein is to be made available at reasonable cost.

Efficiency of transcription and translation (which together comprise expression) is in part dependent upon the nucleotide sequences which are normally situated ahead of the desired coding sequence or gene. These nucleotide sequences or expression control sequences define, inter alia, the location at which RNA polymerase interacts (the promoter sequence) to initiate transcription and at which ribosomes bind and interact with the mRNA (the product of transcription) to initiate translation.

The efficiency with which the resultant mRNA transcript is translated is also often dependent on the stability of the mRNA. The stability of the mRNA is thus quite frequently a rate limiting factor in the production of the desired protein. Stabilizing mRNA can, therefore, result in very significant improvements in production of proteins in host cells. This is particularly the case when the mRNA of a desired product is unstable. Using the methods of the present invention, therefore, production of pharmaceutically important proteins and polypeptides (such as interferon) in host cells (such as E.coli) transformed with DNA encoding those products can be substantially improved by stabilizing the mRNA transcripts for those products. Such proteins can, therefore, be made available to the public at substantially reduced costs.

Processing by RNase III has been shown to alter the stability of mRNA. For example, in the case of the bacteriophage λ int gene, cleavage at the RNase III site by RNase III leads to faster degradation of the mRNA relative to the unprocessed transcript and, thus, lower levels of int protein synthesis (See U. Schmeissner et al., J. Molec. Biol., 176, 39-53 (1984); H. A. Lozeron et al., J. Molec. Biol.,

0204401

109, 359-365 (1977); H. A. Lozeron et al., <u>Virology</u>, <u>71</u>, 262-277 (1976); G. Guarneros et al., <u>Proc. Natl. Acad. Sci. USA</u>, <u>79</u>, 238-242 (1982)). On the other hand, bacteriophage T7 mRNAs, most of which are processed by RNase III, are more stable as a whole than bacterial mRNAs (See B. L. Marrs et al., <u>Nature New Biol.</u>, <u>234</u>, 168-170 (1971)).

<u>Summary of the Invention</u>

The present invention relates to a method of improving the yield of protein produced in a host cell. In the method of the present invention, an RNase III site is inserted downstream of a gene for the desired protein. The site should be such that when said site in mRNA is cleaved by RNase III, a folded structure will remain that will stabilize the mRNA that is being so cleaved to at least some extent relative to mRNA that has not been cleaved. A preferred RNase III site is a bacteriophage T7 site. Most preferred is the bacteriophage T7 R1.1 site.

The present invention also relates to a hybrid DNA comprising a gene coding on expression for a desired protein and DNA coding for an RNase III site that when cleaved by RNase will retain a folded structure on the gene that will stabilize the mRNA that is being cleaved to at least some extent relative to mRNA that has not been cleaved. Such a hybrid DNA may be inserted in an appropriate host, such as E.coli and the <u>E.coli</u>, then cultured to produce the desired protein.

The present invention also relates to a host, such as <u>E.coli</u> (for example, <u>E.coli</u> MC1061) that comprises such a hybrid DNA and to a method of preparing a protein comprising culturing such a host in an appropriate medium.

B.0756

Description Of The Drawings

Figure 1 shows a restriction map of plasmids pCP62 and pCP63.

Figure 2 shows messenger processing in rnc$^{+}$ and rnc$^{-}$ cells. On the autoradiograph shown here, Lane 1: RNA (4µl) extracted from rnc- cells; Lane 2: RNA (4µl) extracted from rnc- cells, Lanes 3-5: [$^{32}$P]- Hae III restriction fragments used as markers. The position and the exact length of the single-stranded markers in nucleotides (nt) are shown on the right. The positions of the unprocessed and processed products are indicated by arrows.

The aforementioned size markers were obtained as follows: [$^{32}$P]-Ml3mp9-590 RFI DNA was digested with Hae III and three bands corresponding to fragments 849, 484, and 341 base pairs in length were eluted and used as size markers without prior strand separation. Alternatively, the 865 nt and the 595 nt [$^{32}$P] single-stranded probes were also used as size markers.

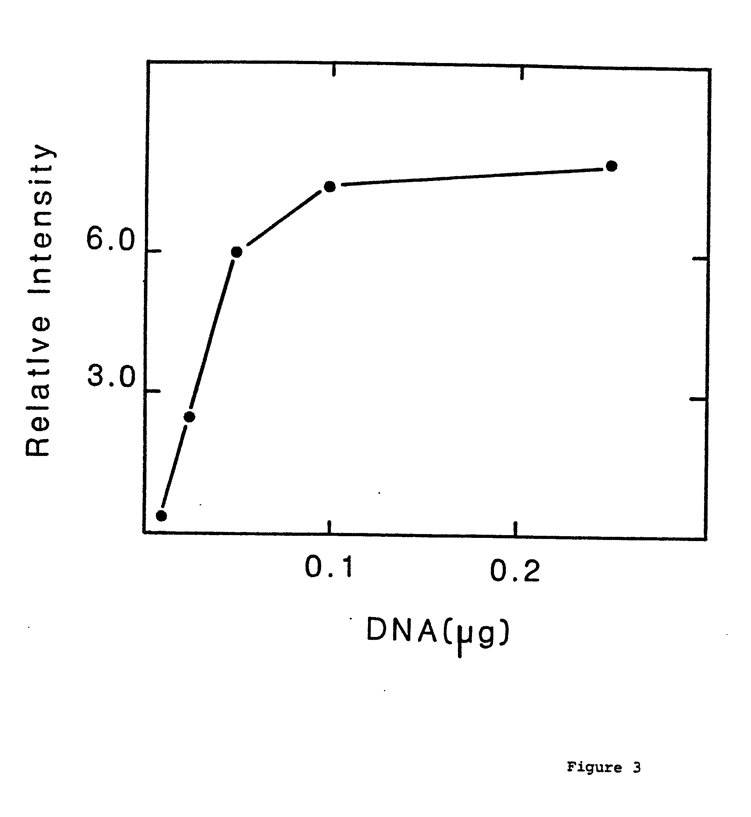

Figure 3 shows a hybridization saturation curve.

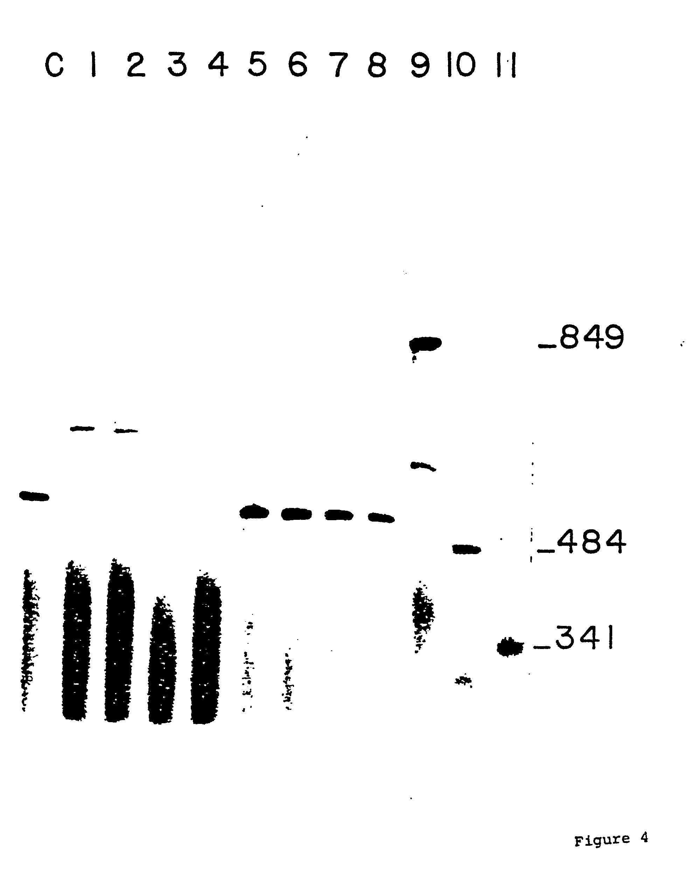

Figure 4 shows decay of full-length and processed mRNAs.

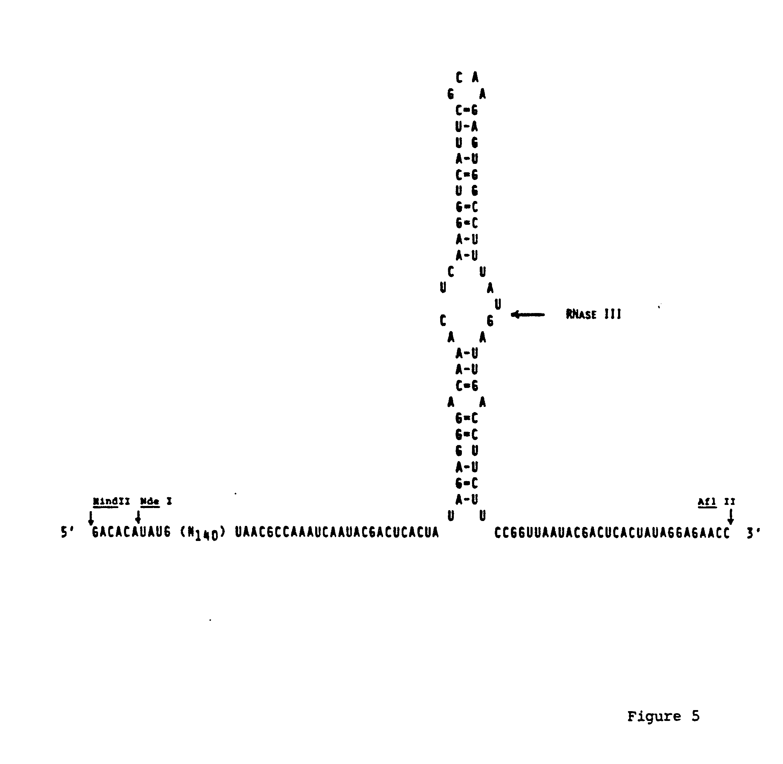

Figure 5 shows the RNA sequence around the RNase III Rl.l site.

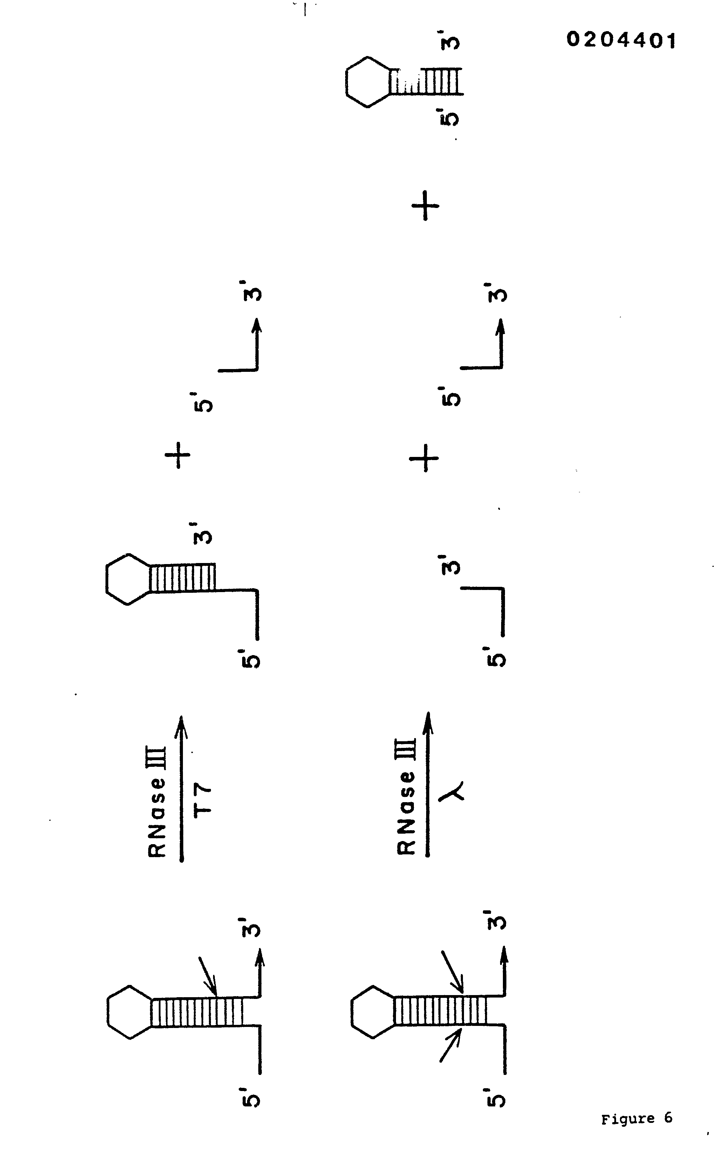

Figure 6 shows single and double cleavage by RNase III.

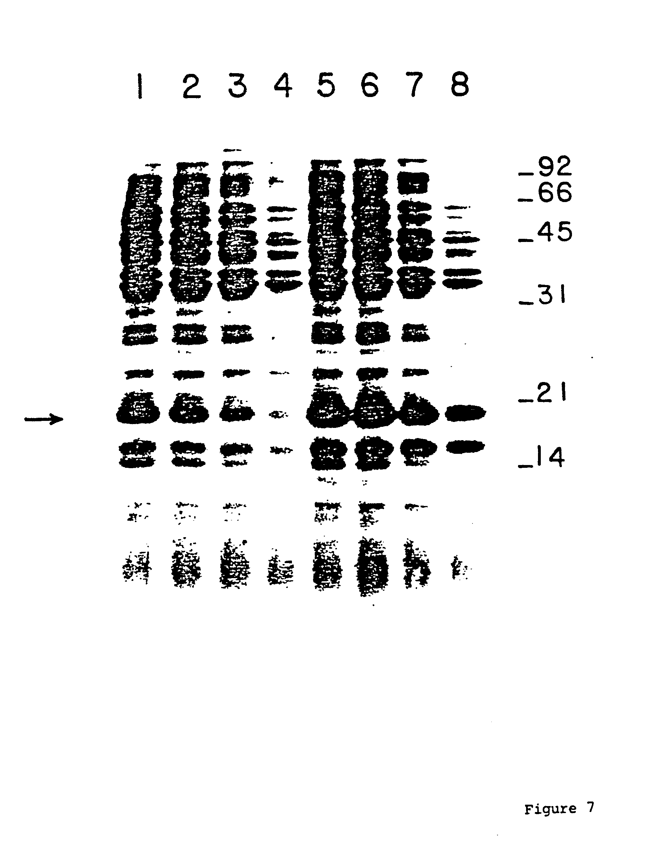

Figure 7 shows protein synthesis after transcription arrest.

Description of the Invention

In accordance with the methods of this invention, an RNase III site is inserted downstream of a gene coding on expression for a desired protein (e.g., adjacent to the 3' end of said gene), in order to stabilize the mRNA transcript. This insertion may be performed before or after the gene is operatively linked to an expression control sequence in an expression vector using any one of many well-known techniques for insertion of DNA sequences such as dG-dC or dA-dT tailing, direct ligation, synthetic linkers, exonuclease and polymerase-linked repair reactions followed by ligation, or extension of the DNA strand with DNA polymerase and an appropriate single-stranded template followed by ligation.

The RNase III site used in the process of this invention should be such that when the mRNA transcript is cleaved by RNase III, a folded structure will be retained by the mRNA transcript for the protein so that such mRNA will be stabilized relative to uncleaved mRNA. One preferred RNase III site is the bacteriophage T7 R1.1 site. However, other RNase III sites that form similar folded structures to that described above may also be used in the process and DNAs of this invention. The particular location of the RNase III site relative to the 3' end of the gene coding for the desired protein is not critical. However, the site must be located at a position such that the mRNA transcript of the gene includes the site and such that upon cleavage by RNase III the cleaved mRNA is stabilized, as described above, as compared to uncleaved mRNA.

As an example of these methods, we cloned a bacteriophage T7 RNase III site downstream of a constitutively expressed foreign gene, and determined the extent of processing, the stability of the cleavage products and the translation efficiency of the mRNA in vivo.

The RNA sequence around the RNase III R1.1 site is shown in Figure 5. The sequence of the transcript through the 261 base pairs HindII - Afl II restriction fragment inserted in pCP63 is drawn in a potential hairpin structure (See J. J. Dunn et al.,
J. Molec. Biol., 166, 477-535 (1983)). The position
of a few restriction sites and the point of cleavage
by RNase III are also shown.

We surprisingly found that processing at a
cloned bacteriophage T7 RNase III site resulted in
strong stabilization of the mRNA relative to the
full-length transcript and that the mode of cleavage
within the RNase III site determined mRNA stability.
For example, single cleavage leaves part of the phage
T7 RNase III site in a folded structure at the generated 3'end and stabilizes the upstream mRNA (coding
for the desired protein) whereas double cleavage,
e.g., at the λ int site, removes the folded structure
and accelerates mRNA degradation.

Single and double cleavage by RNase is
shown in Figure 6. The hairpin structures represent
the secondary structure of the mRNA before and after
processing. The R1.1 site is cleaved once and the
upstream product can maintain a folded structure at
its 3'end which protects the messenger from degradation. All RNase III sites of bacteriophage T7 share
this mode of cleavage and the processed transcripts
are relatively stable. In contrast, the bacteriophage
λ int gene processing site is cleaved twice and the
upstream product which cannot maintain a folded structure at its 3'end becomes labile.

The processed transcript is as active a
messenger as the unprocessed one and can direct protein synthesis for longer times. This increased
efficiency is accompanied by a proportional increase
in protein levels. In fact, in one embodiment of
our invention, we found that processing at the R 1.1
site in pCP63 increased the half-life of the human
interferon α5 mRNA 3 to 4-fold relative to the unprocessed transcript.

The DNA sequences that may advantageously be expressed using the methods of the present invention may be selected from a large variety of DNA sequences that on expression encode prokaryotic or eukaryotic polypeptides. For example, such sequences may encode animal and human hormones, such as any of the various IFN-α's, particularly α2, α5, α6, α7, α8, IFN-β, IFN-γ, human insulin and growth hormones, bovine growth hormone, swine growth hormone and erythropoietin, human blood factors and plasminogen, viral or bacterial antigens, such as the core or surface antigen of HBV or the antigens of FMDV, and other useful polypeptides of prokaryotic or eukaryotic origin. Most preferably, DNA sequences whose mRNA transcripts are unstable and rate limiting are employed.

Methods for expressing these DNA sequences in the expression vectors of this invention and producing the polypeptides coded for by that sequence are well known. They include transforming an appropriate host with an expression vector having the desired DNA sequence operatively-linked to the expression control sequence of the vector, culturing the host under appropriate conditions of growth and collecting the desired polypeptide from the culture. It is most preferred that the host cells be allowed to reach stationary phase before the desired polypeptide is collected. Those of skill in the art may select from known methods those that are most effective for a particular gene expression without departing from the scope of this invention.

In the cloning and expression of the aformentioned DNA sequences a wide variety of vectors are useful. These include, for example, vectors consisting of segments of chromosomal, non-chromosomal and synthetic DNA sequences, such as various known derivatives of SV40, known bacterial plasmids, e.g.,

plasmids from <u>E.coli</u> including col E1, pCR1, pBR322, pMB9 and their derivatives, wider host range plasmids, e.g., RP4, phage DNAs, e.g., the numerous derivatives of phage λ, e.g., NM 989, and other DNA phages, e.g., M13 and <u>filamentous</u> single stranded DNA phages, yeast plasmids such as the 2μ plasmid or derivatives thereof, and vectors derived from combinations of plasmids and phage DNAs, such as plasmids which have been modified to employ phage DNA or other expression control sequences.

Within each specific cloning or expression vehicle, various sites may be selected for insertion of the DNA sequences of this invention. These sites are usually designated by the restriction endonuclease which cuts them and are well recognized by those of skill in the art. Various methods for inserting DNA sequences into these sites to form recombinant DNA molecules are also well known. These include, for example, dG-dC or dA-dT tailing, direct ligation, synthetic linkers, exonuclease and polymerase-linked repair reactions followed by ligation, or extension of the DNA strand with DNA polymerase and an appropriate single-stranded template followed by ligation. It is, of course, to be understood that a cloning or expression vehicle useful in this invention need not have a restriction endonuclease site for insertion of the chosen DNA fragment. Instead, the vehicle could be joined to the fragment by alternative means.

For expression of the aforementioned DNA sequences, these DNA sequences are operatively-linked to one or more expression control sequences in the expression vector. Such operative linking, which may be effected before or after the chosen DNA sequence is inserted into a cloning vehicle, enables the expression control sequences to control and promote the expression of the inserted DNA sequence.

Any of the wide variety of expression control sequences -- sequences that control the expression of a DNA sequence when operatively linked to it -- may be used in these vectors to express the desired DNA sequence. Such useful expression control sequences, include, for example, the early and late promoters of SV40, the lac system, the trp system, the TAC or TRC system, the major operator and promoter regions of phage λ, the control regions of fd coat protein, the promoter for 3-phosphoglycerate kinase or other glycolytic enzymes, the promoters of acid phosphatase, e.g., Pho5, the promoters of the yeast α-mating factors, and other sequences known to control the expression of genes of prokaryotic or eukaryotic cells or their viruses, and various combinations thereof. In mammalian cells, it is additionally possible to amplify the expression units by linking the gene to that coding for dehydrofolate reductase and applying a selection to host Chinese hamster ovary cells.

The vector or expression vehicle, and in particular the sites chosen therein for insertion of the selected DNA fragment and the expression control sequence employed in this invention are determined by a variety of factors, e.g., number of sites susceptible to a particular restriction enzyme, size of the protein to be expressed, expression characteristics such as the location of start and stop codons relative to the vector sequences, and other factors recognized by those of skill in the art. The choice of a vector, expression control sequence, and insertion site for a particular phospholipase inhibitor protein sequence is determined by a balance of these factors, not all selections being equally effective for a given case.

The recombinant DNA molecule containing the desired gene operatively linked to an expression control sequence may then be employed to transform a wide variety of appropriate hosts so as to permit such hosts (transformants) to express the gene, or fragment thereof, and to produce the polypeptide, or portion thereof, for which the hybrid DNA codes. The recombinant DNA molecule may also be employed to transform a host so as to permit that host on replication to produce additional recombinant DNA molecules as a source of desired genes and fragments thereof.

A wide variety of hosts are also useful in the method of this invention. These hosts include, for example, bacteria, such as E.coli (for example, E.coli HB101 or E.coli MC1061), Bacillus, Streptomyces, and Pseudomonas, fungi, such as yeasts, and animal, such as CHO cells, plant cells in tissue culture or other hosts known in the art.

The selection of an appropriate host for either of these uses is controlled by a number of factors recognized by the art. These include, for example, compatibility with the chosen vector, toxicity of the co-products, ease of recovery of the desired polypeptide, expression characteristics, biosafety and costs. No absolute choice of host may be made for a particular recombinant DNA molecule or polypeptide from any of these factors alone. Instead, a balance of these factors must be struck with the realization that not all hosts may be equally effective for expression of a particular recombinant DNA molecule. In the preferred embodiment of this invention, we employed E.coli MC1061 and the plasmid pCP63.

It should be understood that the DNA sequences that are inserted at the selected site of a cloning or expression vehicle may include nucleotides which are not part of the actual gene coding for the desired polypeptide. For example, the DNA sequences may be fused in the same reading frame in an expression vector to a portion of a DNA sequence coding for at least one eukaryotic or prokaryotic carrier protein or a DNA sequence coding for at least one eukaryotic or prokaryotic signal sequence, or combinations thereof. Such constructions may aid in expression of the desired DNA sequence, improve purification or permit secretion, and preferably maturation, of the desired polypeptide from the host cell. The DNA sequence may alternatively include an ATG start codon, alone or together with other codons, fused directly to the sequence encoding the first amino acid of a desired polypeptide. Such constructions enable the production of, for example, a methionyl or other peptidyl polypeptide. This N-terminal methionine or peptide may then, if desired, be cleaved intra- or extra-cellularly by a variety of known processes or the polypeptide used together with the methionine or other fusion attached to it in various compositions and methods.

In order that this invention may be better understood, the following examples, for illustrative purposes only, are described.

## EXAMPLES

### Processing of Plasmid-coded mRNA by RNase III

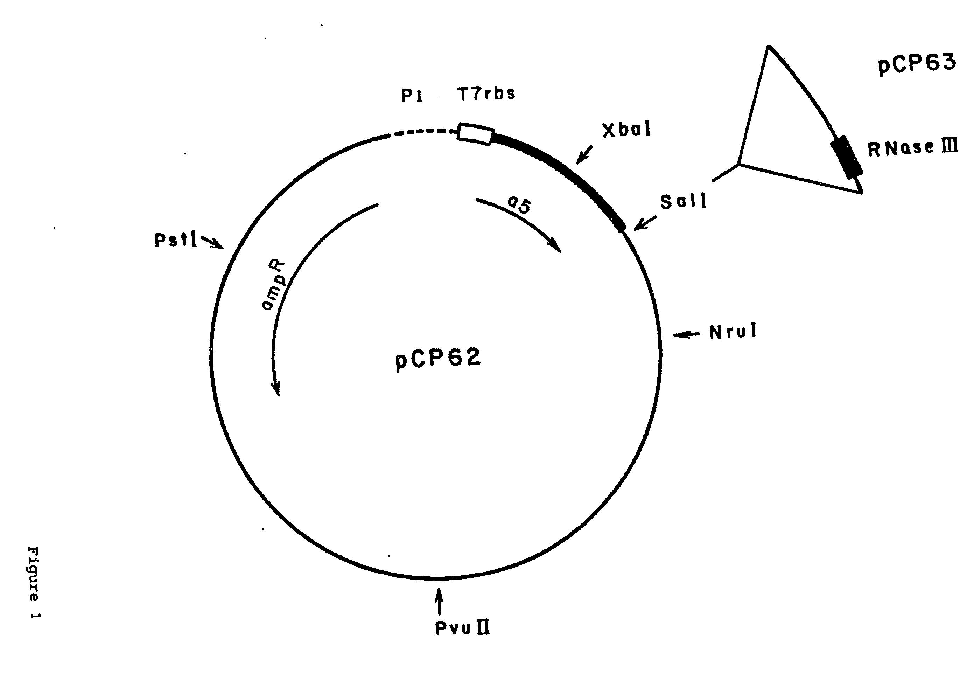

We constructed plasmids pCP62 and pCP63 using standard genetic engineering techniques used in previous studies (see N. Panayotatos, Nucleic Acids Res., 12, 2641-2648 (1984); N. Panayotatos et al., J. Cellular Biochem. Suppl. 7B, 109 (1983); N. Panayotatos et al., Nucleic Acids Res., 9, 5679-5688 (1981); N. Panayotatos et al., J. Biol. Chem., 254, 5555-5561 (1979)). The origin of the various DNA segments constituting each plasmid as well as their exact sequence coordinates are described in Figure 1.

Figure 1 shows a restriction map of pCP62 and pCP63. Plasmid pCP62 (4,617 base pairs), which carries the human interferon α5 gene under the control of a constitutive promoter, consists of 263 base pairs (dotted line) of an EcoRI-EcoRI restriction fragment containing the colEI RNAI promoter (see N. Panayotatos et al., J. Cellular Biochem. Suppl. 7B, 109 (1983)), followed clockwise by a 115 base pairs (open box) EcoRI-Sau3AI restriction fragment from pPI.T7α2 containing the bacteriophage T7 gene 1.1 ribosome binding site (coordinates 5902-6009 of the T7 sequence (see J. J. Dunn et al., J. Molec. Biol., 166, 477-535 (1983)) and the first codon sequence of human leukocyte interferons (See N. Panayotatos et al., J. Cellular Biochem. Suppl. 7B, 109 (1983)), a 516 base pairs Sau3AI-Taq I fragment containing 501 base pairs (solid bar) coding for the human leukocyte interferon α5 sequence and 15 base pairs (solid line) of the downstream chromosomal sequence from plasmid M51α5, and, finally, a 3718 base pairs Sal I-EcoRI restriction fragment containing the replicon region and the β-lactamase gene from pBR322 (see J. G. Sutcliffe, Cold Spring Harbor Symp. Quant. Biol., 43, 77-90 (1979)).

pCP63 (4,861 base pairs) is identical with pCP62 except for a 261 base pairs HincII-Afl II restriction fragment from pRW307 (See N. Panayotatos et al., J. Biol. Chem., 254, 5555-5561 (1979)) containing the bacteriophage T7 RNase III site R1.1 (see H. D. Robertson et al., Proc. Natl. Acad. Sci. USA, 74, 822-826 (1977) and J. J. Dunn J. Mol. Biol. 166, 477-535 (1983)) inserted at the unique Sal I site of pCP62 downstream from the α5 gene (Figure 1).

We generated plasmid pCP63 from plasmid pCP62 by inserting immediately downstream of the 3'-end of the interferon α5 gene, 261 base pairs of the DNA sequence surrounding the bacteriophage T7 RNase III site R1.1 (Figure 1).

To test whether mRNA processing occures at this RNase III site, we prepared single-stranded DNA probes complementary to the mRNA between the Xba I and Nru I sites in pCP62 (592 base pairs) and in pCP63 (865 base pairs) and labelled them with $^{32}$P as follows:

## Single-Stranded Restriction Fragment Probes

We purified a 592 base pairs Xba I- Nru I restriction fragment spanning the Sal I site in pCP62 and the equivalent 865 base pairs Xba I-Nru I fragment spanning the RNase III site in pCP63 (Figure 1) by gel electrophoresis. Filling-in with [$^{32}$P]-labelled deoxynucleoside triphosphates followed by strand separation by gel electrophoresis (See A. M. Maxam et al., Methods Enzymol., 65, 499-559 (1980)) generated anti-sense Xba I-Nru I fragments labelled at the Xba I ends.

## M13 Single-Stranded Probes

We individually cloned the 592 and 865 base pairs Xba I-Nru I fragments from pCP62 and pCP63 at the Sma I site of the M13mp9 vector (see J. Messing et al., Gene, 19, 269-276 (1982)) (JM101 is the host provided with the M13mpg vector and is available from New England Biolabs). We verified the size and identity of the inserts by DNA sequencing and restriction mapping. We prepared (see J. Messing et al., Gene, 19, 269-276 (1982)) phages containing the desired strands (designated M13mp9-592 and M13mp9-865) and stored them at 4°C.

We prepared the M13mp9-256 probe by ligating to the filled-in Hind III site of the M13mp9 vector a 256 base pairs Nde I-Afl II restriction fragment (Figure 5) with filled-in ends. In this

-14-

way, we generated two <u>Hind</u> III sites at the ends of
the insert.

To prepare single-stranded DNA probes, we
infected <u>E.coli</u> JM101 cultures in 2xTY media with
the desired phage at $OD_{590}=1.0$.  Thirty minutes later,
we added $[^{32}P]$-orthophosphate (lmCi/10ml) and we
continued vigorous shaking for 5 h at 37°C.  We
purified phage from the supernatants by two polyethylene glycol precipitations.  We isolated single-
stranded phage DNA from the phage by phenol extraction
and ethanol precipitation (see J. Messing et al.,
<u>Gene</u>, 19, 269-276 (1982)).  We purified double stranded
$[^{32}P]$RFI DNA from the cell pellet of the same cultures
with a quick-lysis procedure (see R. D. Klein et al.,
<u>Plasmid</u>, 3, 88-91 (1980).  We estimated the specific
activity at lmCi/mg DNA with approximate yields of
1 μg RFI DNA and 0.8 μg phage DNA per ml culture.

## S1 Protection

We added rifampicin (0.2 mg/ml) to cultures
growing exponentially ($OD_{590}=0.8-1.0$) and transferred
5 ml samples in tubes containing 2.5 μl diethylpyro-
carbonate kept in ice-salt.  After centrifuging at
4°C, we isolated total RNA from the cell pellet (see
J. Brosius et al., <u>Proc. Natl. Acad. Sci. USA</u>, <u>257</u>,
9205-9210 (1982)) and resuspended in 50 μl $H_2O$.  We
performed hybridization at 65°C, incubation with S1
nuclease at 37°C and electrophoresis on 8% polyacrylamide - 7M urea gels (see J. Brosius et al.,
<u>Proc. Natl. Acad. Sci. USA</u>, <u>257</u>, 9205-9210 (1982)).

## Processing of the mRNA

We prepared total RNA from AO159 rnc- and
AO160 rnc$^+$ cells harboring pCP63, hybridized it to
the $[^{32}P]$-M13mp9-865 single stranded DNA (0.2 μg)
treated with S1 nuclease and electrophoresed on de-
naturing gels.

-15-

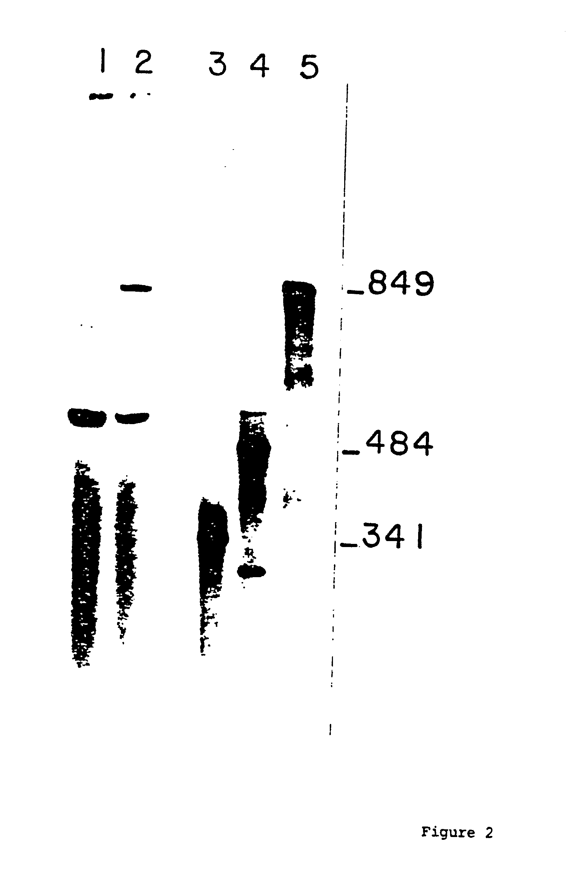

Hybridization of total RNA prepared from RNase III$^+$ (rnc$^+$) AO160 cells harbouring pCP63 to the 865 nt probe protected a 498±20 nt fragment from digestion with S1 nuclease (Figure 2, lane 1). We obtained the same results whether uniformly labelled or 3' end labelled probes were used. The size of the protected fragment corresponded to the product expected if processing occurred at the RNase III site (see H. D. Robertson et al., Proc. Natl. Acad. Sci. USA, 74, 822-826 (1977)).

Processing by RNase III should generate a second fragment of uniformly labelled protected probe from the downstream region between the RNase III and the Nru I sites (367 nt). This fragment is barely visible over the heavier background in that region of the gel (Figure 2, lane 1). We also obtained a weak band (Figure 4, lane C) when we allowed hybridization to proceed at a much lower temperature (37°C rather than 65°C) to discount the possibility that the 367 nt fragment did not hybridize as efficiently as the 498 nt fragment due to its smaller size. The weak intensity of the 367 nt fragment suggested that the mRNA sequence downstream from the RNase III site is degraded faster than the upstream region.

To demonstrate that the cleavage of interferon α5 mRNA at the RNase III site was indeed catalyzed by RNase III, we isolated total RNA from an rnc$^-$ isogenic strain (AO159) harbouring pCP63 (E.coli AO159 rnc- and E.coli AO159 rnc$^+$ are isogenic except for RNase III. Both were obtained from Ursula Schmeissner). After hybridization and digestion by S1 nuclease we obtained two bands corresponding to fragments protected by unprocessed (865 nt) and processed (498 nt) mRNA (Figure 2, lane 2). Considering that the stability of the processed mRNA is 3-4 times higher than that of the unprocessed messenger (see below) an estimated 80% of total messenger is not

-16-

processed in the rnc⁻ host.  The observed processing in these cells may be due to residual levels of RNase III in combination with strong affinity of the enzyme for the R1.1 site on the abundant interferon mRNA.

The fact that in rnc⁺ cells the interferon mRNA is found almost exclusively in the processed form whereas in isogenic rnc⁻ cells it is found mostly in the unprocessed form indicates that the observed processing is the result of RNase III activity at the homologuous site.

To further demonstrate this point, we determined the precise site of cleavage.  We hybridized total mRNA to the M13mp9-256 probe (Figure 5).  We electrophoresed the S1-protected fragment on a DNA sequencing gel along with the four reactions of a known DNA sequence as a marker (data not shown).  We obtained three bands corresponding to DNA fragments 211, 212 and 213 nt long.  The size of these fragments corresponds to the size (212 nt) expected if processing by RNase III occurs at the reported site (see H. D. Robertson et al., _Proc. Natl. Acad. Sci. USA_, 74, 822-826 (1977)).  Therefore, the protected fragments must indeed be the result of precise mRNA processing by RNase III at the R1.1 site.

A major difficulty in studying mRNA cleavage is that putative "processing" sites prove occasionally to be "leaky" transcriptional terminators. This does not seem to be the case here.  The T7 RNase III R1.1 site under study is a well characterized processing site both _in vivo_ and _in vitro_ (see H. D. Robertson et al., _Proc. Natl. Acad. Sci. USA_, 74, 822-826 (1977); M. Rosenberg et al., in "Processing of RNA" (J. J. Dunn, ed.) pp. 277-285, Brookhaven National Laboratory, Upton, New York (1975)) which does not act as a rho-dependent terminator (see J. J. Dunn et al., _Proc. Natl. Acad. Sci. USA_, 70, 1559-1563 (1973)). The observed reduction in the extent of processing in the rnc⁻ strain as well as the fact that the cleavage site of the interferon α5 mRNA coincides with that of T7 mRNA processed in vivo strongly argue that the protected 498 nt and 367 nt fragments are produced after processing of the mRNA by RNase III. The differential intensities of the bands corresponding to the protected segments of mRNA upstream and downstream of the RNase III site must, therefore, be attributed to differential stability of the cleavage products.

## Processing by RNase III Increases the Half-life of the mRNA

Before determining the degradation rate of mRNA, it was necessary to ensure that the amount of probe was not merely in excess, but actually at saturating concentrations, so that a decrease in the amount of mRNA would be accompanied by a corresponding decrease in the amount of protected probe.

We prepared total mRNA from E.coli MC1061 rnc⁺ cells harboring pCP62 or pCP63 and we incubated the volume of total RNA (4µl) used in the previous experiments with increasing amounts of the M13mp9-592 and the M13mp9-865 probes, respectively. After digesting with S1 nuclease and gel electrophoresis, we measured the band intensities of the protected probe and plotted them as a function of DNA concentration. The relative intensities of the bands were determined by scanning the autoradiograph in a Shimadzu CS930 spectrophotometer. From the hybridization saturation curve shown in Figure 3 we concluded that incubation of 4µl of total mRNA with 0.2 µg of the M13mp9-865 probe ensured saturating probe conditions. We obtained similar curves with total RNA prepared from cells harbouring pCP62 and hybridized with the M13mp9-592 probe (data not shown).

To determine the half-life of mRNA in vivo, we blocked transcription initiation with rifampicin and we performed S1-protection experiments under saturating concentrations of probe. After blocking transcription initiation by the addition of rifampicin to exponentially growing cultures, we hybridized total RNA prepared at the indicated times from MC1061 cells harboring pCP62 to the [$^{32}$P]-M13mp9-592 probe (Figure 4, lanes 1-4) and we hybridized total RNA prepared from MC1061 cells harboring pCP63 to the [$^{32}$P]-M13mp9-865 probe (Figure 4, lanes 5-8). The size markers described in Figure 2 were loaded on lanes 9-11 (Figure 4). A sample hybridized at 37°C was loaded on lane C (Figure 4). An autoradiograph of samples removed at 0 min (lanes 1, 5); 2 min (lanes 2, 6); 5 min (lanes 3, 7); and 8 min (lanes 4, 8) is shown Figure 4. The positions of the unprocessed and processed products are indicated by arrows.

Figure 4 shows that a band corresponding to the full length of the 592 nt probe which does not contain the RNase III site is protected from S1 nuclease. The intensity of this band begins to decrease immediately after the addition of rifampicin and less than 20% remains 5 min later. In contrast, the intensity of the 498 nt probe generated from the sequence upstream of the RNase III site appears to increase 2 min after the addition of rifampicin and 90% of the probe is still protected at 5 min. (The initial increase in intensity has been observed with other long-lived mRNAs (See A. Von Gabain et al., Proc. Natl. Acad. Sci. USA, 80, 653-657 (1983))). Semi-logarithmic plots of band intensity versus time showed that the unprocessed mRNA decays with a half-life of 2-1/2 min, whereas the mRNA fragment generated by RNase III decays with a half-lie of approximately 9 min. Thus, processing RNase III resulted in a 3-4 fold increase in the stability of the mRNA segment upstream from the cleavage site.

However, only the upstream fragment of mRNA became more stable. The downstream part appeared to be degraded with a half-life similar to that of the unprocessed transctipt, although the weak intensity of the corresponding band did not permit accurate measurements. Since this mRNA cleavage product and the unprocessed transcript terminate at the same 3'-ends their half-lives would be expected to be the same, if mRNA degradation occurred primarily exonucleolytically from the 3'end.

By analogy, the unprocessed mRNA and the processed fragment upstream from the RNase III site share the same 5'ends and, accordingly, the increased stability of the latter should be attributed to increased resistance to exonucleolytic degradation from the 3'end. Evidence for 3'-to-5' directionality in mRNA decay was also obtained in a recent study (See A. Von Gabain et al., Proc. Natl. Acad. Sci. USA, 80, 653-657 (1983)).

## Geometry of Cleavage Determines Stability

Processing at the bacteriophage T7 RNase III R1.1 site generates an upstream mRNA fragment which maintains at its 3'end a portion of the hairpin structure of the RNase III site, and a downstream fragment whose newly generated 5' end sequence loses the potential of being involved in a double stranded structure. This feature is a consequence of the particular location of the cleavage point relative to the loop of the proposed secondary structure for the R1.1 site (Figure 5).

Folding of the 3'end in a secondary structure is believed to protect the RNA from 3'-to-5' exonucleolytic degradation. Therefore, the observed 3-4 fold increase in stability of the processed upstream fragment may be attributed to the fact that
its 3' end is protected.  In this case, processing
at other RNase III sites sharing the same geometry
of cleavage should also result in more stable processed mRNAs.

## Processed mRNA is Active Messenger in vivo

We found that processing at the R1.1 site
in pCP63 increased the half-life of the human inter-
feron $\alpha$5 mRNA 3-4 fold relative to the unprocessed
transcript.  If the processed transcript were as
efficient a messenger as the unprocessed one then,
after blocking mRNA synthesis with rifampicin, protein
synthesis catalyzed by the decaying mRNA should be
prolonged proportionally to the half-lives of the
messengers.

Addition of rifampicin to exponentially
growing cultures of MC1061 cells harbouring pCP62 or
pCP63 leads to mRNA decay with the kinetics shown in
Figure 4.  To determine the efficiency of the surviving mRNA molecules for protein synthesis, we grew
cultures overnight in L-broth and diluted ten fold
with M9 media supplemented with 0.2% glucose,
1 mg/liter thiamine and the usual amino acids except
cysteine at 10 mg/liter.  After 4 hrs at 30°C, the
cultures reached mid-exponential phase ($OD_{590}$=1.0).
We added rifampicin (0.2 mg/ml) and at various times
afterwards [$^{35}$S]-cysteine (0.03 mCi/ml).  Ten minutes
after adding [$^{35}$S]-cysteine, we added L-cysteine
(0.4 mg/ml) and we chilled the cultures quickly in
an ice/salt bath.  We processed cells from 1 ml culture and analyzed them by gel electrophoresis (See
U. K. Laemmli, Nature, 277,680-685 (1970)).  Visualization of labelled proteins by autoradiography after
gel electrophoresis showed that the decrease of interferon 5 synthesis after transcription arrest is strikingly similar to the mRNA decay.  Lanes 1-4 of Figure 7 show pCP62/MC1061 cells labelled at 0, 3, 6 and 10 min after the addition of rifampicin. Lanes 5-8 of Figure 7 show pCP63/MC1061 cells labelled in parallel.

Interferon synthesis catalyzed by unprocessed mRNA in cells harbouring pCP62 begins to decrease 2 min after the addition of rifampicin and decays with a half-life of approximately 2-1/2 min. On the other hand, interferon synthesis catalyzed by processed mRNA in cells harbouring pCP63 shows the same small increase as the mRNA 2 min after addition of rifampicin and subsequently decays with a half-life of approximately 9 min (Figure 7). The fact that the decrease in interferon synthesis closely parallels the decay of mRNA demonstrates that the processed mRNA is as efficient a template for protein synthesis as the unprocessed one.

In addition, since the processed mRNA can function as active messenger 3-4 times longer, the levels of human interferon α5 protein are also increased. This increase can be observed in Figure 7 by comparing the band intensities of α5 interferon in cells harbouring pCP62 and pCP63. Moreover, measurements of interferon α5 acitvity in cells harbouring pCP63 revealed 3-4 times higher levels of active interferon than in cells harbouring pCP62 (data not shown). These results demonstrate that RNase III acts, in this case, as a positive modulator of gene expression.

It should also be noted that the protected single-stranded probe fragments were visible on the polyacrylamide gels after staining with ethidum bromide. This observation (which may eliminate the use of labelled probe in future experiments) suggested that the intracellular levels of interferon mRNA were quite high. If a visible band roughly represents 10 ng of the 498 nt fragment (20% of total at saturating

proce concentrations) then the number of α5 interferon
mRNA molecules per cell is calculated at 200.

Despite the fact that the interferon mRNA
levels are approaching those of the most abudant
bacterial mRNA (see A. Hirashima et al., Proc.
Natl. Acad. Sci. USA, 71, 4149-4153 (1974)), we found
processing by RNase III to be complete. This result
suggested that RNase III is present in the cell in
large excess. Therefore, small variations in its
levels are not likely to determine the extent of
processing. Instead, the key element to processing
appears to be the presence of an RNase III site downstream from the gene, and the main determinant of
the fate of the processed transcript appears to be
the location of the cleavage point within that site.
Between the level of mRNA lability conferred by
λ int-like double cleavage and the stabilization provided by T7-like single cleavage, the half-life of
the messenger can be altered by more than an order
of magnitude. Within this range, the affinity of
the particular RNase III site for the enzyme may
determine the precise degree of messenger stability.

While we have hereinbefore described a
number of embodiments of this invention, it is
apparent that our basic constructions can be altered
to provide other embodiments which utilize the processes and compositions of this invention. Therefore,
it will be appreciated that the scope of this invention is to be defined by the claims appended hereto
rather than by the specific embodiments which have
been presented hereinbefore by way of example.

CLAIMS

1.  A method of improving the yield of a desired polypeptide produced in a host cell comprising culturing a host cell transformed with a DNA sequence coding on expression for that protein, said DNA sequence also comprising an RNase III site downstream of said coding sequence, said site being such that when said site is cleaved by RNase III, a folded structure remains that stabilizes the mRNA transcript for the desired protein relative to uncleaved mRNA.

2.  A method according to claim 1 wherein said RNase III site is a bacteriophage T7 site.

3.  A method according to claim 2 wherein the RNase III site is the T7 R1.1 site.

4.  A method according to claim 1, wherein said host is selected from the group consistng of strains of E.coli, Pseudomonas, Bacillus, yeasts, or other fungi, mouse or other animal or plant hosts and human tissue cells.

5.  A method according to claim 4 wherein said host is E.coli.

6.  A polypeptide produced by the method of any one of claims 1 to 6.

7.  The polypeptide according to claim 6, selected from the group of human and animal lymphokines, growth hormones, blood factors, plasminogens, and viral and bacterial antigens.

8.  A method of stabilizing mRNA comprising inserting an RNase III site downstream of a DNA sequence coding on expression for a desired protein, said site being such that when said site in mRNA is cleaved by RNase III, a folded structure remains that stabilizes the mRNA relative to uncleaved mRNA.

9.  A method according to claim 8, wherein the RNase III site is a bacteriophage T7 site.

10.    A method according to claim 9, wherein the RNase III site is the T7 Rl.1 site.

11.    A recombinant DNA comprising a DNA sequence coding on expression for a desired protein and an RNase III site downstream of said coding sequence, said site being such that when said site in mRNA is cleaved by RNase III, a folded structure remains that stabilizes the mRNA relative to uncleaved mRNA.

12.    A DNA according to claim 11, wherein the RNase III site is the bacteriophage T7 site.

13.    A DNA according to claim 12, wherein the RNase III site is the T7 Rl.1 site.

14.    A DNA according to claim 11, also including an expression control sequence operatively linked to the DNA sequence coding for the desired protein.

15.    A DNA according to claim 14, wherein said expression control sequence is selected from the group consisting of the early and late promoters SV 40, the lac system, the TAC system, the TRC system, the trp system, major operator and promotor regions of phage λ, the control regions of fd coat protein, the promoter for 3-phosphoglycerate kinase or other glycolytic enzymes, the promoters of acid phosphatase, the promoters of yeast α-mating factors, and other sequences which control the expression of genes of prokaryotic or eukaryotic cells or their viruses.

16.    A host transformed with the DNA of claim 14.

17.    A host transformed with the DNA of claim 15.

18.    The transformed host according to claim 16, selected from the group consisting of strains of E.coli, Pseudomonas, Bacillus, yeasts, or other fungi, mouse, swine or other animal or plant hosts and human tissue cells.

19.    The transformed host according to claim 17, selected from the group consisting of strains of E.coli, Pseudomonas, Bacillus, yeasts, or other fungi, mouse, swine or other animal or plant hosts and human tissue cells.

20.    The transformed host according to claim 18, wherein said host is E.coli.

21.    The transformed host according to claim 19, wherein said host is E.coli.

Figure 1

0204401

Figure 2

Figure 3

Figure 4

```
                              C A
                              G   A
                              C-G
                              U-A
                              U G
                              A-U
                              C-G
                              U G
                              G-C
                              G-C
                              A-U
                              A-U
                           C     U
                          U        A
                                    U
                          C        G  ←———— RNASE III
                          A        A
                              A-U
                              A-U
                              C-G
                           A     A
                              G-C
                              G-C
                              G U
                              A-U
                              G-C
                              A-U
    HindII  Nde I                                              Afl II
       ↓     ↓                U   U                              ↓
5'  GACACAUAUG (N₁₄₀) UAACGCCAAAUCAAUACGACUCACUA    CCGGUUAAUACGACUCACUAUAGGAGAACC  3'
```

Figure 5

Figure 6

Figure 7

| | DOCUMENTS CONSIDERED TO BE RELEVANT | | EP 86302560.7 | |
|---|---|---|---|---|
| Category | Citation of document with indication, where appropriate, of relevant passages | Relevant to claim | CLASSIFICATION OF THE APPLICATION (Int. Cl.4) | |
| A | EP - A2 - 0 075 444 (GENENTECH)<br><br>* Claims 1,7-10; page 27, lines 5,6; abstract *<br><br>-- | 1,4,5, 8,11, 14-18, 20 | C 12 N 15/00<br>C 12 P 21/00<br>C 07 K 15/00<br>C 07 K 13/00<br>C 07 H 21/04<br>C 12 N 1/20<br>C 12 N 1/16<br>C 12 N 1/14<br>C 12 N 5/00<br>// C 12 R 1:19<br>C 12 R 1:38<br>C 12 R 1:07 | |
| D,A | NATURE NEW BIOLOGY, vol. 234, no. 44, November 3, 1971, London<br><br>B.L. MARRS et al. "Host and Bacteriophage Specific Messenger RNA Degradation in T7-Infected Escherichia coli"<br>pages 168-170<br><br>* Totality *<br><br>-- | 1-5,8-10 | | |
| D,A | JOURNAL OF MOLECULAR BIOLOGY, vol. 176, no. 1, June 15, 1984, London, ...<br><br>U. SCHMEISSNER et al. "Removal of a Terminator Structure by RNA Processing Regulates int Gene Expression"<br>pages 39-53<br><br>* Page 51, line 36 - page 52, line 4 *<br><br>---- | 1-5,8-10 | **TECHNICAL FIELDS SEARCHED (Int. Cl.4)**<br><br>C 12 N<br>C 12 P<br>C 07 K<br>C 07 H | |

The present search report has been drawn up for all claims

| Place of search | Date of completion of the search | Examiner |
|---|---|---|
| VIENNA | 16-07-1986 | FARNIOK |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82